# EUROPEAN PATENT APPLICATION

(11) **EP 2 138 096 A1**
(43) Date of publication of application: **30.12.2009**
(21) Application number: 09161742.3
(22) Date of filing: 03.06.2009
(51) Int. Cl.: A61B 5/083, A61B 5/087, A61B 5/097, A61M 16/10

(54) **Flow measurement system and biological information monitor**

(30) Priority: 03.06.2008 JP 2008145893
(71) Applicant: Nihon Kohden Corporation, Shinjuku-ku Tokyo (JP)
(72) Inventor: Takhashi, Iwao, Tokyo (JP); Utsunomiya, Hidetaka, Tokyo (JP); Hosaka, Shoichi, Tokyo (JP); Takatori, Fumihiko, Tokyo (JP); Matsubara, Isao, Tokyo (JP)
(74) Representative: Grünecker, Kinkeldey, Stockmair & Schwanhäusser Anwaltssozietät

(57) **Abstract**

A flow measurement system includes: a respiration monitoring apparatus which measures a flow of a gas flowing through a flow path, to output a measurement value; and a gas monitoring apparatus which samples the gas to measure at least one of a concentration of a component of the gas and a component of the gas and to generate information on the flow of the sampled gas. The measurement value is corrected based on the information.

## Description

### BACKGROUND OF THE INVENTION

The present invention relates to a flow measurement system and biological information monitor which are to be used for measuring, for example, the volume of ventilation of a patient during use of an artificial respirator (anesthesia apparatus) for an operation or the like, and the concentration and/or component of a gas such as carbon dioxide, oxygen, or anesthesia gas.

A related-art flow measurement system which is to be used for measuring, for example, the volume of ventilation of the patient during use of an artificial respirator (anesthesia apparatus) for an operation or the like, and the concentration and/or component of anesthesia gas will be described.

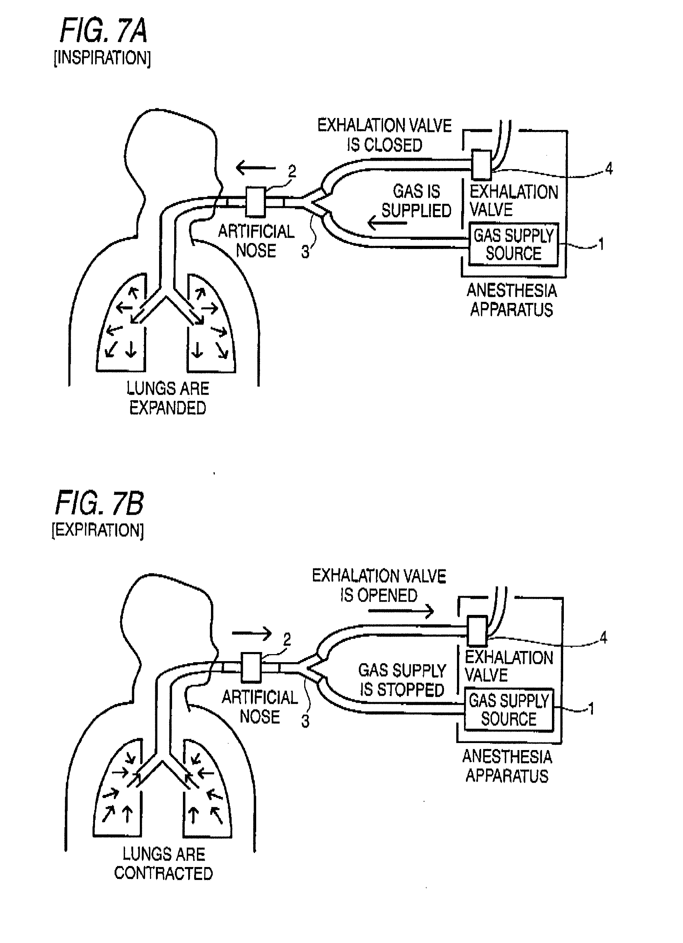

Figs. 7A to 7D show the basic concept of a flow measurement of anesthesia gas.

As shown in Figs. 7A and 7B, artificial respiration to a patient includes an inspiratory phase and an expiratory phase.

An artificial respirator (anesthesia apparatus) has a supply source 1 of a gas containing an anesthetic component, and supplies the gas to the patient through a corrugated tube 3 to feed the gas into the lungs of the patient (during anesthesia, the patient cannot spontaneously respire, and hence the gas is pressurizedly supplied in a state where an exhalation valve 4 shown in the figure is closed).

During the expiratory phase, the gas supply is stopped, and the exhalation valve 4 is opened, so that the gas is exhaled through the exhalation valve 4 by contraction of the lungs.

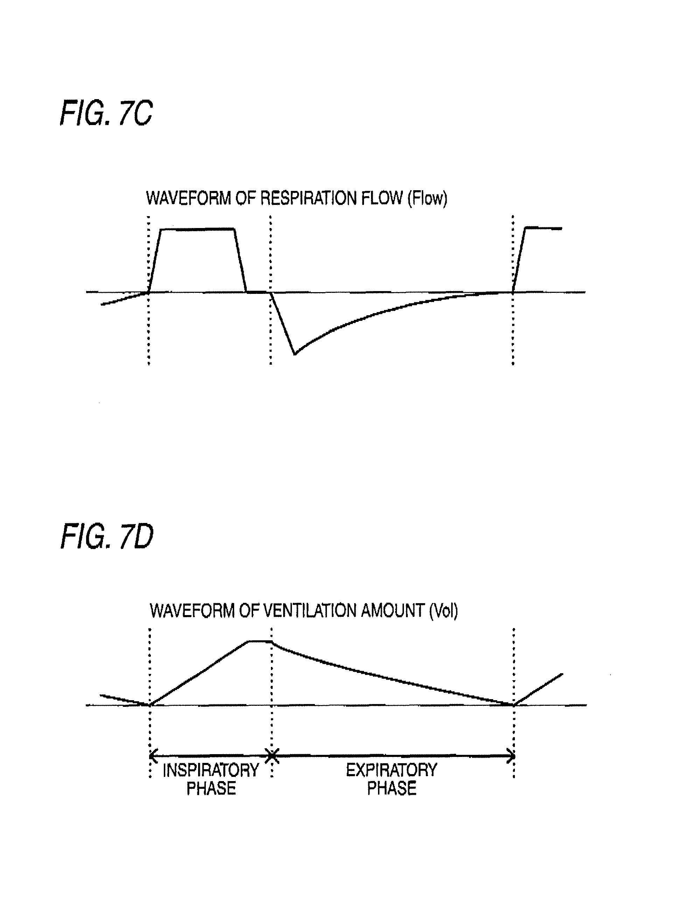

Figs. 7C and 7D show waveforms of the respiration flow and the volume of ventilation in this state.

Fig. 7C is a waveform chart in which the ordinate indicates the instantaneous value of the flow during inspiration and expiration, and the abscissa indicates the time (sec). Fig. 7D is a waveform chart in which the ordinate indicates an integrated value of the flow during inspiration and expiration, and the abscissa indicates the time (sec). In Fig. 7C, inspiration is indicated by (+), and expiration is indicated by (-).

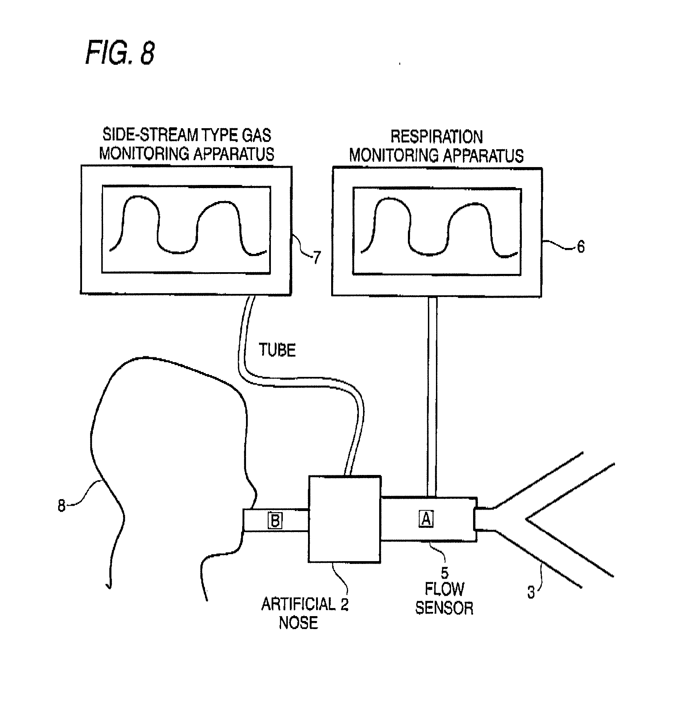

Fig. 8 shows a first related-art example of a flow measurement system which measures, for example, the volume of ventilation of the patient during use of an artificial respirator (anesthesia apparatus) for an operation or the like, and the concentration and/or component of anesthesia gas.

Fig. 8 shows the example which is disclosed in U.S. Pat. No. 5,379,650, and in which a flow sensor having no sampling port is singly used, and a sampling point for a gas monitoring apparatus is between a respiration monitoring apparatus and the patient.

Referring to Fig. 8, anesthesia gas from a gas supply source of the anesthesia apparatus which is not shown is supplied from a corrugated tube 3 to the patient 8 through the flow sensor 5 and an artificial nose 2 functioning as a humidifier.

In the flow sensor 5, a pair of measuring narrow tubes which are formed at the both ends of an orifice forming member are connected to an input of the respiration monitoring apparatus 6.

A measuring narrow tube extending from a sampling port disposed in the artificial nose 2 is connected to an input of the gas monitoring apparatus 7 through a tube.

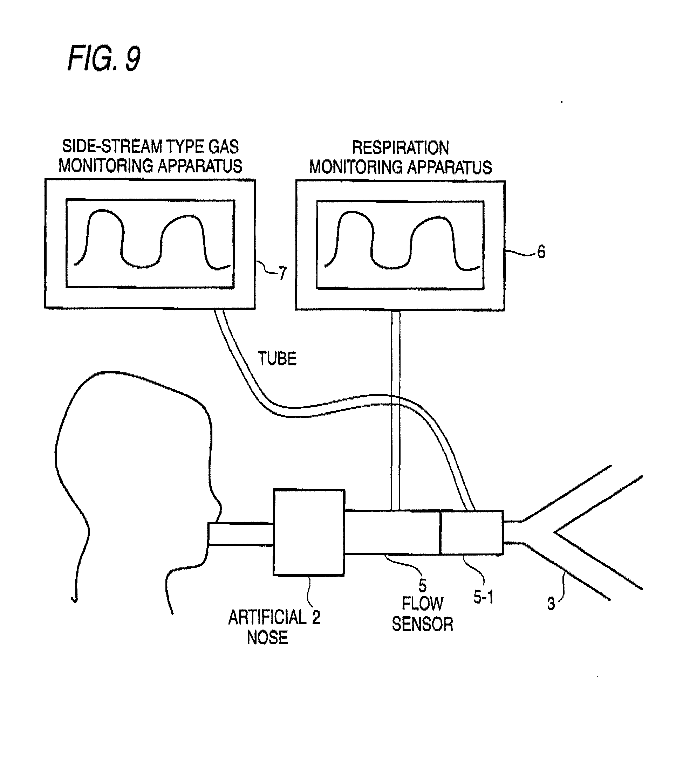

Fig. 9 shows a second related-art example of a flow measurement system which measures, for example, the volume of ventilation of the patient during use of an artificial respirator (anesthesia apparatus) for an operation or the like, and the concentration and/or component of anesthesia gas.

Fig. 9 shows the example in which a flow sensor 5 and a gas sampling port 5-1 are integrated with each other, and a sampling point for a gas monitoring apparatus is between a respiration monitoring apparatus 6 and the artificial respirator (anesthesia apparatus) that is not shown.

Referring to Fig. 9, anesthesia gas from a gas supply source of the anesthesia apparatus is supplied from a corrugated tube 3 to a gas monitoring apparatus 7 through the gas sampling port 5-1 which is formed integrally with the artificial-respirator side of the flow sensor 5.

The flow sensor 5 is connected to a patient 8 through an artificial nose 2 functioning as a humidifier.

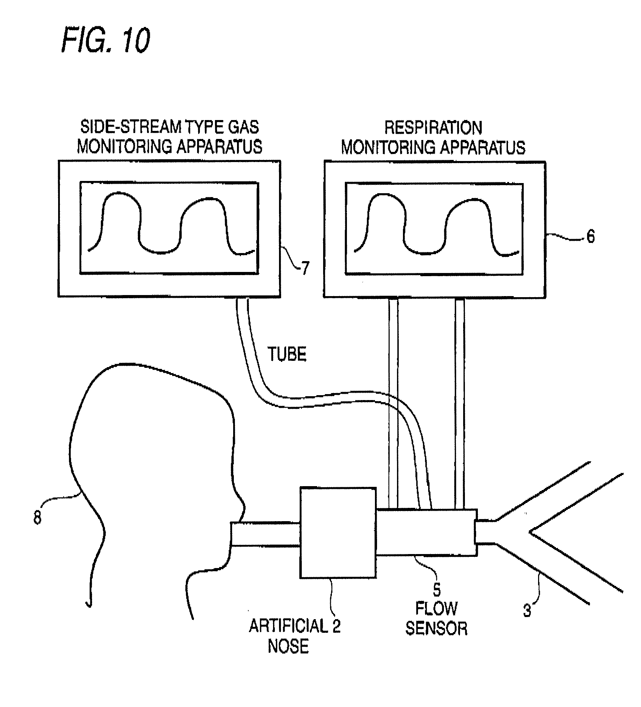

Fig. 10 shows a third related-art example of a flow measurement system which measures, for example, the volume of ventilation of the patient during use of an artificial respirator (anesthesia apparatus) for an operation or the like, and the concentration and/or component of anesthesia gas.

Fig. 10 shows the example in which, as disclosed in U.S. Pat. No. 5, 088, 332, a flow sensor 5 and a gas sampling port are integrated with each other, and a sampling port for a gas monitoring apparatus is between a pair of measuring narrow tubes which are formed at the both ends of an orifice forming member of the flow sensor.

Referring to Fig. 10, anesthesia gas from a gas supply source of the anesthesia apparatus is supplied from a corrugated tube 3 to the gas monitoring apparatus 7 through the gas sampling port which is formed integrally with the flow sensor 5.

The flow sensor 5 is connected to a patient 8 through an artificial nose 2 functioning as a humidifier.

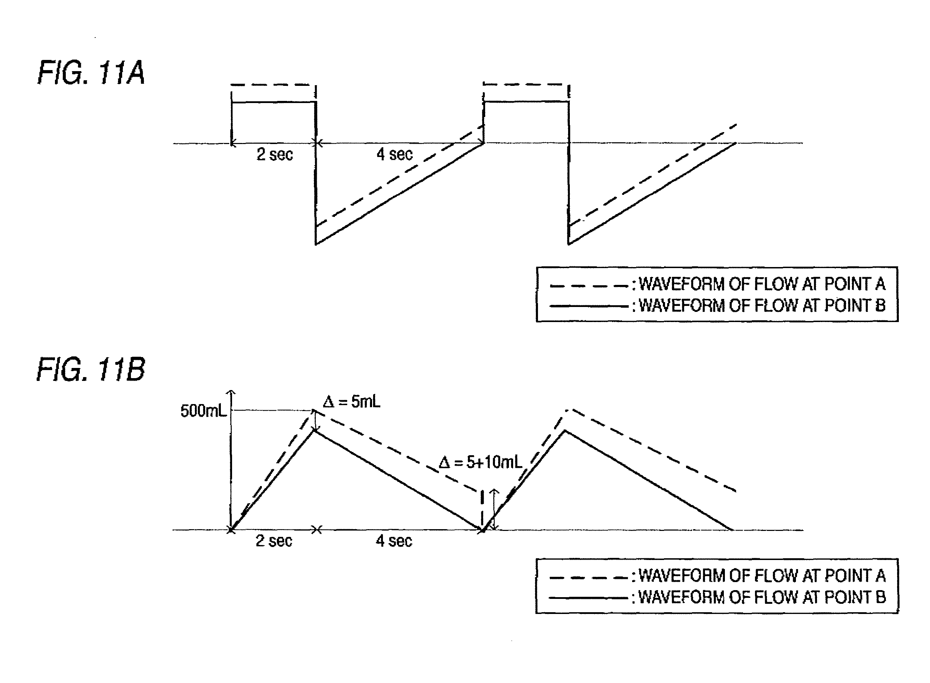

Problems of the related-art example shown in Fig. 8 will be described with reference to Figs. 11A and 11B.

For example, the volume of ventilation of the artificial respirator (anesthesia apparatus) is set to 500 mL, the respiratory rate to 10 times/min., I : E (Inspiration time : Expiration time) to 1 : 2, and the volume of sampling to 150 mL/min.

Fig. 11A is a waveform chart which corresponds to Fig. 7C, and in which the ordinate indicates the instantaneous value of the flow during inspiration and expiration, and the abscissa indicates the time (sec). In Fig. 11A, inspiration is indicated by (+), and expiration is indicated by (-).

In Fig. 11A, the broken line indicates the instantaneous value of the flow at point A of Fig. 8, and the solid line indicates the instantaneous value of the flow at point B. The figure shows that inspiration for 2 seconds and expiration for 4 seconds are repeated, and the sampling to the gas monitoring apparatus causes the flow at B which is the actual volume of ventilation of the patient, to be reduced as compared to that at A which is the flow measured by the respiration monitoring apparatus.

Fig. 11B is a waveform chart which corresponds to Fig. 7D, and in which the ordinate indicates an integrated value of the flow during inspiration and expiration, and the abscissa indicates the time (sec).

In Fig. 11B, the solid line indicates the integrated value of the flow at point B of Fig. 8 (the volume which is actually inspired and expired by the patient) (in the inspiratory phase, positive integration, and, in the expiratory phase, negative integration). When inspiration for 2 seconds and expiration for 4 seconds are repeated, the integrated flow at point B reaches 495 mL as indicated by the solid line, for 2 seconds which are the time period of the inspiratory phase, and returns to zero which is the control point, for the next expiratory phase or 4 seconds.

The broken line of Fig. 11B indicates the integrated value of the flow at point A of Fig. 8 (in the inspiratory phase, a positive integrated value, and, in the expiratory phase, a negative integrated value). When inspiration for 2 seconds and expiration for 4 seconds are repeated, the integrated flow at point A reaches 500 mL as indicated by the broken line, for 2 seconds which are the time period of the inspiratory phase, and returns to a point that is larger by 15 mL than zero which is the control point, for the next expiratory phase or 4 seconds. When the next respiration is started, the integrated value is reset to zero.

The difference of 15 mL corresponds to the volume of the gas which is sampled during one respiration cycle.

In the flow measurement system of Fig. 8, namely, there is a problem in that the value measured by the respiration monitoring apparatus 6 is larger by the sampled volume (in Fig. 11B, 15 mL) than the actual volume of ventilation of the patient, and the volume of ventilation of the patient due to actual inspiration and expiration cannot be correctly measured.

In the second related-art example of Fig. 9, the sampling point for the gas monitoring apparatus is placed in front of the flow sensor which measures the gas flow, and the flow of the gas which has been sampled is measured. Therefore, the problem in that the value measured by the respiration monitoring apparatus 6 is larger by the sampled volume than the actual volume of ventilation of the patient is not caused.

In the second related-art example, however, an element for forming the sampling point for the gas monitoring apparatus in front of the flow sensor is required, and hence there occurs a problem in that the conduit line length is prolonged and the volume of the dead space is increased.

In the third related-art example of Fig. 10, the sampling to the gas monitoring apparatus is performed between the both ends of the orifice forming member, and hence there is a problem in that the orifice forming member affects the flow versus differential pressure characteristics, and the flow of gas cannot be correctly measured.

### SUMMARY

It is therefore an object of the invention to provide a flow measurement system which can measure a correct volume of ventilation of a patient without being affected by the volume of sampling of a gas monitoring apparatus and increasing the volume of the dead space in the system. It is another object of the invention to provide a biological information monitor which can measure a correct volume of ventilation of a patient without being affected by the volume of sampling of a gas monitoring apparatus.

In order to achieve the object, according to the invention, there is provided a flow measurement system comprising:
a respiration monitoring apparatus which measures a flow of a gas flowing through a flow path, to output a measurement value; and
a gas monitoring apparatus which samples the gas to measure at least one of a concentration of a component of the gas and a component of the gas and to generate information on the flow of the sampled gas,
wherein the measurement value is corrected based on the information.

The flow measurement system may further include: a biological information monitor on which the corrected measurement value is displayed in a form of a numerical value or a waveform.

The gas monitoring apparatus may sample the gas by a side stream method.

The respiration monitoring apparatus and the gas monitoring apparatus may be integrated as a module with the biological information monitor.

In order to achieve the object, according to the invention, there is also provided a biological information monitor comprising:
a respiration measurement module which measures a flow of a gas flowing through a flow path, to output a measurement value;
a gas measurement module which samples the gas to measure at least one of a concentration of a component of the gas and a component of the gas and to generate information on the flow of the sampled gas; and
a displaying portion on which a value, which corresponds to the measurement value corrected based on the information, is displayed in a form of a numerical value or a waveform.

The gas measurement module and the respiration measurement module may be integrated with each other.

The biological information monitor may further include:
a correcting unit for correcting the measurement value based on the information.

The correcting unit may be provided in the respiration measurement module or the gas measurement module.

### BRIEF DESCRIPTION OF THE DRAWINGS

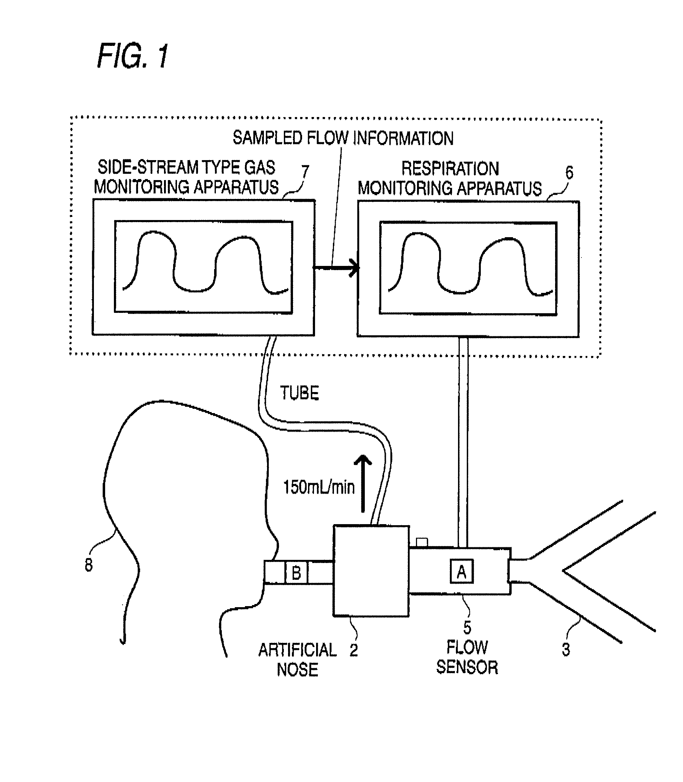

Fig. 1 is a view showing a first embodiment of the flow measurement system of the invention.

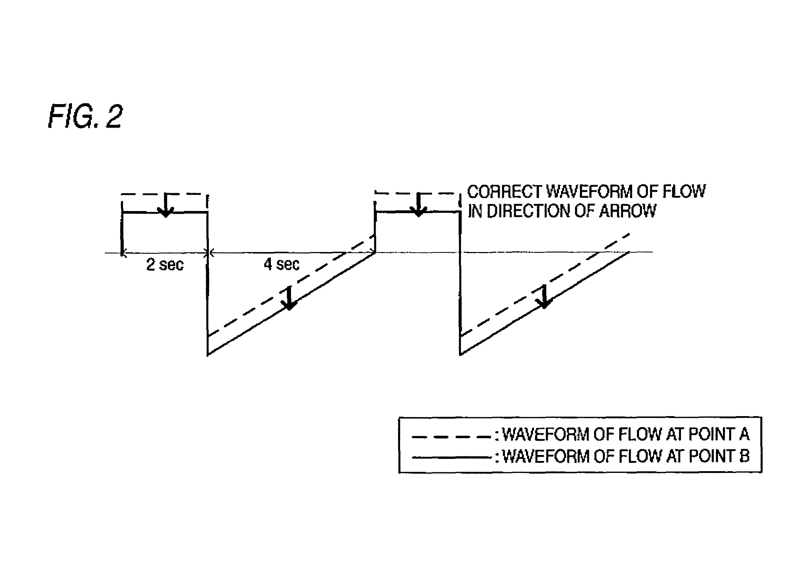

Fig. 2 is a view illustrating that the actual volume of ventilation of the patient is measured while a measurement value of a respiration monitoring apparatus is corrected on the basis of sampled flow information obtained in a gas monitoring apparatus in the invention.

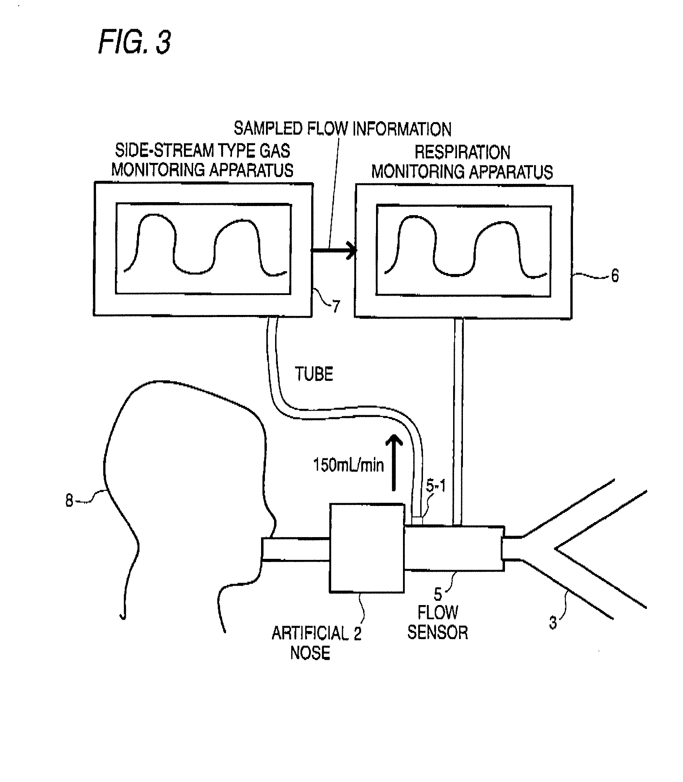

Fig. 3 is a view showing a second embodiment of the flow measurement system of the invention.

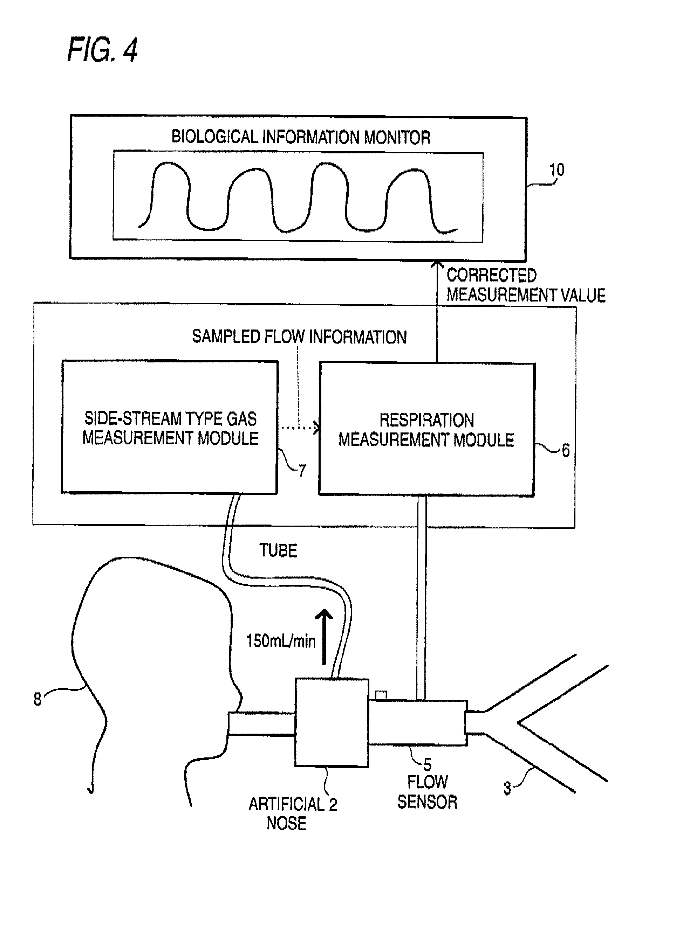

Fig. 4 is a view showing a third embodiment of the flow measurement system of the invention.

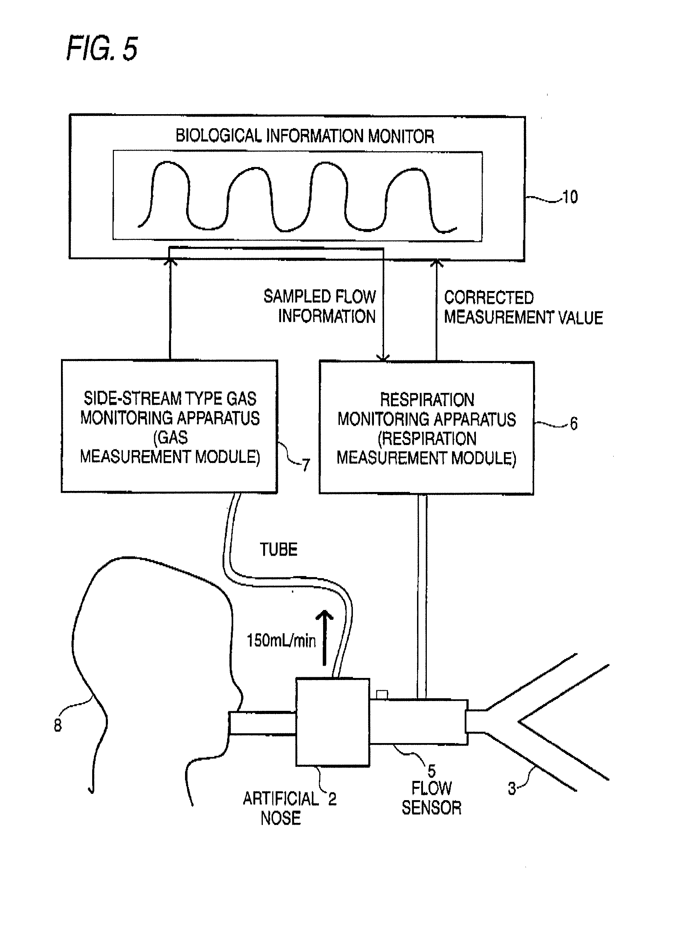

Fig. 5 is a view showing a fourth embodiment of the flow measurement system of the invention.

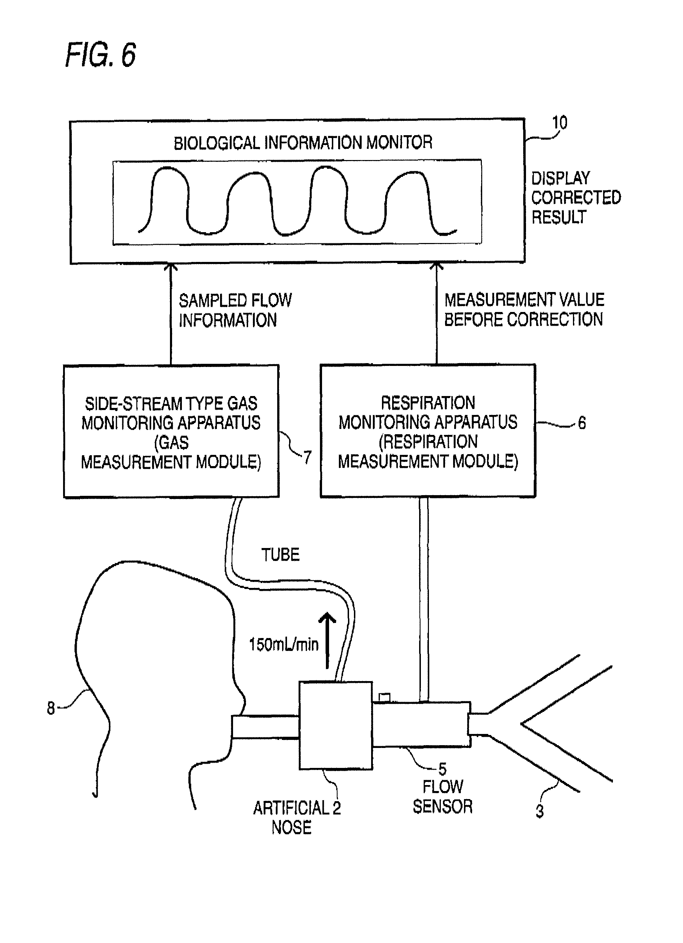

Fig. 6 is a view showing a fifth embodiment of the flow measurement system of the invention.
Figs. 7A to 7D are views showing the basic concept of a flow measurement of anesthesia gas.
Fig. 8 is a view showing a first related-art example of a flow measurement system.
Fig. 9 is a view showing a second related-art example of a flow measurement system.
Fig. 10 is a view showing a third related-art example of a flow measurement system.
Figs. 11A and 11B are views illustrating problems of the first related-art example of Fig. 8.

### DETAILED DESCRIPTION OF PREFERRED EMBODIMENTS

Fig. 1 shows a first embodiment of the flow measurement system of the invention which is to be used for measuring, for example, the volume of ventilation of a patient during use of an artificial respirator (anesthesia apparatus) for an operation or the like, and the concentration and/or component of anesthesia gas.

In the embodiment, a sampling point for a gas monitoring apparatus 7 which can measure anesthesia gas, carbon dioxide, oxygen, or the like is formed in an artificial nose 2 that is between a respiration monitoring apparatus 6 and a patient 8.

Referring to Fig. 1, anesthesia gas from a gas supply source of the anesthesia apparatus which is not shown is supplied from a corrugated tube 3 to the patient 8 through a flow sensor 5 and the artificial nose 2 functioning as a humidifier.

In the flow sensor 5, a resistance portion for generating a differential pressure is formed, and a pair of measuring narrow tubes which are formed at the both ends of the resistance portion are connected to the respiration monitoring apparatus 6.

A measuring narrow tube (sampling tube) extending from the sampling port disposed in the artificial nose 2 is connected to the gas monitoring apparatus 7.

In Fig. 1, information of a sampled flow obtained in the gas monitoring apparatus 7 is supplied to the respiration monitoring apparatus 6 to be used in correction of a flow measurement value in the respiration monitoring apparatus 6.

Fig. 1 shows the example in which the respiration monitoring apparatus 6 and gas monitoring apparatus 7 that are enclosed by the broken line are integrated with each other. It is a matter of course that the respiration monitoring apparatus 6 and the gas monitoring apparatus 7 may be configured so that the sampled flow information in the gas monitoring apparatus 7 is supplied to the respiration monitoring apparatus 6.

Fig. 2 is a view illustrating that the actual volume of ventilation of the patient is measured while the measurement value of the respiration monitoring apparatus 6 is corrected on the basis of the sampled flow information obtained in the gas monitoring apparatus 7 in the invention.

Fig. 2 is a waveform chart which corresponds to Fig. 11A, in which the ordinate indicates the instantaneous value of the flow during inspiration and expiration, and the abscissa indicates the time (sec), and which shows flows measured in points A and B in the flow measurement system of Fig. 1. In the figured, inspiration is indicated by (+), and expiration is indicated by (-).

In the embodiment, the broken line indicates the instantaneous value of the flow at point A of Fig. 1, the solid line indicates the instantaneous value of the flow at point B, and inspiration for 2 seconds and expiration for 4 seconds are repeated. As a result of the sampling to the gas monitoring apparatus, the flow at B which is the actual volume of ventilation of the patient is smaller than that at A which is the flow measured by the respiration monitoring apparatus. As shown in Fig. 1, however, the sampled flow information obtained in the gas monitoring apparatus 7 is supplied to the respiration monitoring apparatus 6, and the value measured by the respiration monitoring apparatus is corrected as indicated by the arrows in Fig. 2. This correction enables measurement which is equal to the actual volume of ventilation of the patient, to be performed.

In the first embodiment of the invention, therefore, it is possible to realize a flow measurement system which can measure a correct volume of ventilation of the patient without being affected by the volume of sampling of the gas monitoring apparatus and increasing the volume of the dead space in the system.

Next, Fig. 3 shows a second embodiment of the flow measurement system of the invention.

In the embodiment, the sampling port which guides a gas to the gas monitoring apparatus 7 is formed integrally with the patient side of the flow sensor 5. Furthermore, anesthesia gas from the gas supply source of the anesthesia apparatus which is not shown is supplied from the corrugated tube 3 to the patient 8 through the flow sensor 5.

In the flow sensor 5, the resistance portion for generating a differential pressure is formed, and the pair of measuring narrow tubes which are formed at the both ends of the resistance portion are connected to the respiration monitoring apparatus 6.

The measuring narrow tube (sampling tube) extending from a sampling port 5-1 formed in the patient side of the flow sensor 5 is connected to the gas monitoring apparatus 7.

In the embodiment, the configuration where the sampling port for the gas monitoring apparatus 7 is formed integrally with the flow sensor enables the volume of the dead space to be reduced.

Next, Fig. 4 shows a third embodiment of the flow measurement system of the invention.

In the embodiment, a sampling point for a gas measurement module 7 which can measure anesthesia gas, carbon dioxide, oxygen, or the like is formed in the artificial nose 2 that is between a respiration measurement module 6 and the patient 8. The gas measurement module 7 and the respiration measurement module 6 are integrated with each other and incorporated in a biological information monitor.

Referring to Fig. 4, anesthesia gas from a gas supply source of the anesthesia apparatus which is not shown is supplied from the corrugated tube 3 to the patient 8 through the flow sensor 5 and the artificial nose 2 functioning as a humidifier.

In the flow sensor 5, the resistance portion for generating a differential pressure is formed, and the pair of measuring narrow tubes which are formed at the both ends of the resistance portion are connected to the respiration measurement module 6.

The measuring narrow tube (sampling tube) extending from the sampling port disposed in the artificial nose 2 is connected to the gas measurement module 7. In this case, the sampling tube may be connected to the sampling port for the flow sensor 5.

In Fig. 4, information of a sampled flow obtained in the gas measurement module 7 is supplied to the respiration measurement module 6 to be used in correction of a flow measurement value in the respiration measurement module 6. The corrected measurement value is supplied to the biological information monitor 10, and output on a screen of the biological information monitor 10, as the corrected measurement value or waveform.

Fig. 2 is a view illustrating that the actual volume of ventilation of the patient is measured while the measurement value of the respiration measurement module 6 is corrected on the basis of the sampled flow information obtained in the gas measurement module 7 in the invention. The contents of the figure have been described in the first embodiment as described above, and hence the description of the contents is omitted.

Next, Fig. 5 shows a fourth embodiment of the flow measurement system of the invention.

In the embodiment, the sampling point for the gas monitoring apparatus 7 which can measure anesthesia gas, carbon dioxide, oxygen, or the like is formed in the artificial nose 2 that is between the respiration monitoring apparatus 6 and the patient 8.

Referring to Fig. 5, anesthesia gas from a gas supply source of the anesthesia apparatus which is not shown is supplied from the corrugated tube 3 to the patient 8 through the flow sensor 5 and the artificial nose 2 functioning as a humidifier.

In the flow sensor 5, the resistance portion for generating a differential pressure is formed, and the pair of measuring narrow tubes which are formed at the both ends of the resistance portion are connected to the respiration monitoring apparatus 6.

The measuring narrow tube (sampling tube) extending from a sampling port formed in the artificial nose 2 is connected to the gas monitoring apparatus 7. In this case, the sampling tube may be connected to the sampling port for the flow sensor 5.

In Fig. 5, information of a sampled flow obtained in the gas monitoring apparatus 7 is supplied to the respiration monitoring apparatus 6 through the biological information monitor 10 to be used in correction of a flow measurement value in a CPU (not shown) in the respiration monitoring apparatus 6. The corrected measurement value or waveform is output on the screen of the biological information monitor 10. The respiration monitoring apparatus 6 and the gas monitoring apparatus are configured as independent apparatuses. Alternatively, the apparatuses may be configured as a module, and incorporated in the biological information monitor 10.

Fig. 2 is a view illustrating that the actual volume of ventilation of the patient is measured while the measurement value of the respiration monitoring apparatus 6 is corrected on the basis of the sampled flow information obtained in the gas monitoring apparatus 7 in the invention. The contents of the figure have been described in the first embodiment as described above, and hence the description of the contents is omitted.

Next, Fig. 6 shows a fifth embodiment of the flow measurement system of the invention.

In the embodiment, the sampling point for the gas monitoring apparatus 7 which can measure anesthesia gas, carbon dioxide, oxygen, or the like is formed in the artificial nose 2 that is between the respiration monitoring apparatus 6 and the patient 8.

Referring to Fig. 6, anesthesia gas from a gas supply source of the anesthesia apparatus which is not shown is supplied from the corrugated tube 3 to the patient 8 through the flow sensor 5 and the artificial nose 2 functioning as a humidifier.

In the flow sensor 5, the resistance portion for generating a differential pressure is formed, and the pair of measuring narrow tubes which are formed at the both ends of the resistance portion are connected to the respiration monitoring apparatus 6.

The measuring narrow tube (sampling tube) extending from a sampling port formed in the artificial nose 2 is connected to the gas monitoring apparatus 7. In this case, the sampling tube may be connected to the sampling port for the flow sensor 5.

In Fig. 6, information of a sampled flow obtained in the gas monitoring apparatus 7 and the flow measurement value obtained in the respiration monitoring apparatus 6 are supplied to the biological information monitor 10, and the flow measurement value is corrected by a CPU (not shown) in the monitor 10. The corrected measurement value or waveform is output on the screen of the biological information monitor 10.

Fig. 2 is a view illustrating that the actual volume of ventilation of the patient is measured while the measurement value of the respiration monitoring apparatus 6 is corrected by the biological information monitor 10 on the basis of the sampled flow information obtained in the gas monitoring apparatus 7 in the invention. The contents of the figure have been described in the first embodiment as described above, and hence the description of the contents is omitted.

Although, in the above, the preferred embodiments of the invention have been described, the invention is not restricted to the above-described embodiments. For example, the portion where the flow measurement value is corrected is not restricted to the places of the embodiments. The sampled flow information may be supplied to the flow sensor, respiration monitoring apparatus (respiration measurement module) or gas monitoring apparatus (gas measurement module), which includes a CPU, and the correction of the flow measurement value may be performed in both the sensor and the apparatus. Various modifications may be performed without departing from the spirit of the invention. For example, the flow sensor is not restricted to the differential type, and may be of the hot-wire type or the ultrasonic type, and, although the configuration where anesthesia gas is used as the gas to be sampled has been disclosed, the invention may be applied also to carbon dioxide, oxygen gas, and the like.

According to an aspect of the invention, it is possible to realize a flow measurement system which can measure a correct volume of ventilation of a patient without being affected by the volume of sampling of the gas monitoring apparatus and increasing the volume of the dead space in the system.

According to an aspect of the invention, it is possible to realize a biological information monitor which can measure a correct volume of ventilation of a patient without being affected by the volume of sampling of a gas monitoring apparatus.

## Claims

1. A flow measurement system comprising:
a respiration monitoring apparatus which measures a flow of a gas flowing through a flow path, to output a measurement value; and
a gas monitoring apparatus which samples the gas to measure at least one of a concentration of a component of the gas and a component of the gas and to generate information on the flow of the sampled gas,
wherein the measurement value is corrected based on the information.

2. The flow measurement system according to claim 1 further comprising: a biological information monitor on which the corrected measurement value is displayed in a form of a numerical value or a waveform.

3. The flow measurement system according to claim 1, wherein the gas monitoring apparatus samples the gas by a side stream method.

4. The flow measurement system according to claim 2, wherein the respiration monitoring apparatus and the gas monitoring apparatus are integrated as a module with the biological information monitor.

5. A biological information monitor comprising:
a respiration measurement module which measures a flow of a gas flowing through a flow path, to output a measurement value;
a gas measurement module which samples the gas to measure at least one of a concentration of a component of the gas and a component of the gas and to generate information on the flow of the sampled gas; and
a displaying portion on which a value, which corresponds to the measurement value corrected based on the information, is displayed in a form of a numerical value or a waveform.

6. The biological information monitor according to claim 5, wherein the gas measurement module and the respiration measurement module are integrated with each other.

7. The biological information monitor according to claim 5, further comprising: a correcting unit for correcting the measurement value based on the information.

8. The biological information monitor according to claim 7, the correcting unit is provided in the respiration measurement module or the gas measurement module.
